(19) Europäisches Patentamt
European Patent Office
Office européen des brevets

(11) Publication number: **0 337 081 A1**

(12) **EUROPEAN PATENT APPLICATION**

(21) Application number: 89102827.6

(51) Int. Cl.4: G01N 33/569 , G01N 33/552

(22) Date of filing: 18.02.89

(30) Priority: 31.03.88 US 175550
06.10.88 US 254329

(43) Date of publication of application:
18.10.89 Bulletin 89/42

(84) Designated Contracting States:
CH DE ES FR GB IT LI

(71) Applicant: BIO-RAD LABORATORIES, INC.
1000 Alfred Nobel Drive
Hercules California 94547(US)

(72) Inventor: Dechene, Neal E.
4 Arete Court
Hercules, California 94547(US)
Inventor: Rathburn, Barbara J.
6410 Conlin Avenue
El Cerrito, California 94530(US)
Inventor: Regan, Barbara P.
2 Guerrero Street, No.208
San Francisco, California 94103(US)
Inventor: Walker, Roger P.
861 Oxford Way
Benecia, California 94510(US)

(74) Representative: Patentanwälte Dipl. Ing. R. Splanemann Dr. B. Reitzner Dipl. Ing. K. Baronetzky
Tal 13
D-8000 München 2(DE)

(54) **Biospecific assay using a derivatized glass surface.**

(57) A biospecific assay method where detection of herpesviruses using silane-derivatized solid supports is disclosed. The solid supports are silanized to produce surface aminoalkyl, alkyl, or aminophenoxy groups, whereby adsorption of herpes virus particles onto the derivatized solid support is substantially increased. The method involves disrupting a herpesvirus in a sample with a solubilization agent, immobilizing the herpesvirus so disrupted on the derivatized solid support, contacting the immobilized herpesvirus with a primary antibody specific for the disrupted virus particle to form an immunocomplex, contacting the immunocomplex with a second antibody which has a label appended thereto to form a labeled immunocomplex, and detecting the labeled immunocomplex.

EP 0 337 081 A1

# BIOSPECIFIC ASSAY USING A DERIVATIZED GLASS SURFACE

## BACKGROUND OF THE INVENTION

The present invention relates generally to assay methods. In particular, the invention relates to biospecific assays for detection of herpesviruses (or herpesviridae) using a derivatized solid support.

To date, there is lacking a simple, quick and effective method for rapid detection of herpesviruses in biological fluids. Isolating and identifying viruses, such as herpes simplex virus (HSV), from biological specimens typically requires several days to weeks. Moreover, the usual procedures cannot be readily performed in most clinical laboratories, and therefore, involve added handling of the specimen. Generally, most diagnoses depend on retrospective tests (e.g., serologic examination of acute and convalescent area). More rapid diagnosis is provided by procedures such as immunoassays, such as, for example, ELISA, or immunofluorescence microscopy; however, such techniques lack sensitivity and require confirmation with culture. With the more serious infections, rapid, accurate detection of the virus becomes crucial and often lifesaving.

Several assays are currently available for detecting particular types of microorganisms. Bucher, U.S. Patent No. 4,588,680 (PCT July 31, 1981) discloses an immunoassay for detection of influenza virus. Immunoassays for the detection of Neisseria gonorrhoeae is disclosed in Abram, U S. Patent No 4,497,900 (March 28, 1983). Armstrong, U.S. Patent No. 4,497,899 (March 28, 1983) discloses an immunoassay for detecting Chlamydia bacteria. Each of the preceding immunoassays uses untreated polystyrene as the solid support. Robertson, PCT WO-86/02733 (October 31, 1985) discloses a sandwich assay for detection of viral antigens associated with ophthalmic disorders.

Weetall, in "Porous Glass For Affinity Chromatography," Methods in Enzymology, 34: 59-72 (1974), discloses silanized porous glass useful for affinity chromatography. Silanization of controlled-pore glass, as disclosed in Weetall, is to attach chemical groups to a glass column and to prevent specific attraction of proteins onto the glass columns, rather than to bind virus antigen onto a solid support as disclosed in the present invention.

In sum, the currently available assays for detecting herpesviruses and the like are expensive, they are often inaccurate, time-consuming, and require undue handling. There is thus a need for a method of detecting herpesviruses in an efficient, quick and economical way with high sensitivity and without further confirmation.

## SUMMARY OF THE INVENTION

The present invention provides a biospecific assay method for herpesvirus using a silane-derivatized solid support. A highly sensitive method of detection for a specific binding reaction between a virus antigen and a complementary binding member is achieved by use of a solid support treated with a silanizing agent, thereby increasing the total amount of virus antigen adsorbed. More specifically, the present invention encompasses the following method for detecting herpesvirus antigen in a sample comprising: disrupting herpesvirus in a sample, immobilizing the herpesvirus so disrupted on a silane-derivatized solid support, contacting the immobilized herpesvirus with a primary antibody specific for the disrupted virus particle to form an immunocomplex, contacting the immunocomplex with a second antibody which has a label appended thereto to form a labeled immunocomplex, and detecting the labeled immunocomplex.

Surprisingly, the derivatization of a solid support by silanization is found to increase the adsorption of herpes virus antigen, particularly herpes simplex antigen, on the solid support. The affinity of this adsorption is sufficient, such that it can replace conventional capture antibody. The solid support is selected in accordance with the availability of surface hydroxyl groups on the solid support surface, which allows it to be derivatized by silanization.

The present invention is applicable to a wide range of biospecific assays. The invention is useful with the more traditional labels used in radioimmunoassay, enzyme immunoassay, fluorescence immunoassay. amd DNA probe assay, as well as, with time-resolved fluorescence immunoassay.

Among the many advantages provided by the instant invention are the ease, accuracy and inexpensiveness of this detection method. Other advantages, aspects, and embodiments of the invention will be apparent from the following description.

## DETAILED DESCRIPTION OF THE INVENTION AND PREFERRED EMBODIMENTS

It is to be understood that although the following description and examples are specifically directed to herpes simplex virus (HSV), the present invention contemplates as useful all herpesviruses herein described. Examples of these viruses include varicellazoster, herpes simplex virus type I & II, cytomegalovirus, and Epstein-Barr virus. Among these viruses, herpes simplex virus type I & II are preferred.

In accordance with the novel methods of the present invention, a sample infected with HSV is obtained using conventional medical and microbiological techniques. The sample thus obtained contains HSV antigen to be determined. To increase the sensitivity of the assay, it is preferred to disrupt the HSV and expose epitopes on the HSV surface, thereby increasing the number of antigenic sites available for binding to HSV antibodies. Conventional techniques that may be employed to disrupt the virus include, for example, treatment with a solubilizing agent, preferably a chaotropic agent; and most preferably a detergent, such as, for example, a nonionic detergent.

Additionally, to maximize detection sensitivity, the solid surface for capture of the subject antigen is derivatized using a silanizing agent, thereby permitting non-immunological binding to the solid phase. The combination of providing for more reactive sites on the antigen, while increasing the adsorption of these disrupted viral particles on the solid supports, creates a new method for detecting HSV.

Derivatization of the glass support using a silanizing agent such as, for example, an aminoalkyltrialkoxysilane, or a halotrialkylsilane, may be performed, for example, in the manners described hereinbelow. Among the aminoalkyltrialkoxysilanes, 3-aminopropyltriethoxysilane is preferred ("3-aminopropyl"); even more preferred, is γ-(p-aminophenoxy)propyltriethoxysilane ("p-aminophenyl"). Among the halotrialkylsilanes. chlorodimethylisopropylsilane ("chlorodimethyl") is preferred.

Thus, a 0.1% 3-aminopropyltriethoxysilane (v/v) solution in dicholoromethane is combined with sodalime glass beads (Sigma) at a ratio of 1.0 mL silane solution per one gram of beads. The beads are stirred for approximately two hours at room temperature. After the silane solution has been decanted, the beads are stirred in one volume of methanol (equal to the volume of silane solution) for five minutes and the solution is decanted. A second volume of methanol is used to rinse the beads and decanted. The beads are then dried in a vacuum desiccator for approximately one hour.

Similar HSV results have been produced when 3-aminopropyl silanized borosilicate glass test tubes or silanized glass fiber filters were used instead of the sodalime glass beads. Specifically, 12 x 75 mm borosilicate glass tubes were silanized by adding 1 ml of 0.1% 3-aminopropyltriethoxysilane (v/v) solution in $CH_2Cl_2$ to each tube. The tubes were vertically rotated for 2 hours and the silane solution was decanted. After dispensing 1 ml of methanol into each tube, the tubes were rotated for 5 minutes and decanted. A second volume of methanol was used to rinse the tubes. The tubes were decanted and "air dried" for approximately 2 hours.

Likewise, improved detection of HSV has been obtained when borosilicate glass tubes were independently silanized with chlorodimethylisopropylsilane and γ-(p-aminophenoxy)propyltriethoxysilane. The method used for obtaining these results is substantially identical to the method, previously described, for 3-aminopropyl silanized borosilicate glass tubes, distinguished only by the particular silanizing agent used.

Moreover, similar HSV results were produced when sodalime glass tubes were independently silanized with 3-aminopropyltriethoxysilane, chlorodimethylisopropylsilane, and γ-(p-aminophenoxy)-propyltriethoxysilane. Again, the method used for obtaining these results is substantially identical to the method, previously described, for 3-aminopropyl silanized borosilicate glass tubes, distinguished only by the use of sodalime glass tubes as the solid support material and the particular silanizing agent used.

Assays performed on a quaternary amine glass surface have also been found useful in the present invention. The quaternary amine glass surface can be prepared by, for example, incubating 3-aminopropyl glass in a 1% iodomethane ($CH_3I$) solution.

In a preferred embodiment of the present invention, HSV is added to a protein medium before being pipetted into the assay. The protein medium functions as the antigen transport medium. Suitable protein media include, for example, Minimum Essential Medium (MEM) containing 2% fetal calf serum or Hanks Balanced Salt Solution containing 0.5% gelatin. It is preferred to employ MEM (Gibco) containing 2% fetal calf serum as the protein medium.

In accordance with the present invention, the sample is subjected to lysing conditions to lyse (or "disrupt") the virus and expose HSV antigen for immobilization on the solid support. The lysing conditions may be any conventional conditions or procedures known to result in lysing the virus. A preferred method is the use of solubilization agents. Illustrative of suitable solubilization agents useful for disrupting the virus

include, for example, nonionic detergents, zwitterionic detergents, and chaotropic agents, such as, for example, urea, potassium thiocyanate and guanidine HCl.

Examples of nonionic detergents useful in the present invention include: Octylphenol-ethylene oxide condensate (Nonidet P-40® (NP-40)), Polyoxyethylenesorbitan monolaurate (Tween 20®), Octylphenoxy polyethoxyethanol (Triton X-100®), and N-octyl-β-D-glucopyranoside. Among these nonionic detergents, NP-40® is preferred. Examples of zwitterionic detergents include, for example, N-tetradecyl-N,N-dimethyl-3-ammonio-1-propanesulfonate (Zwittergent 3-14®) and 3-[(3-cholamidopropyl)dimethylammonio]-1-propanesulfonate (CHAPS). The addition of any of these solubilizing agents to the protein medium produces superior sensitivity and specificity with respect to the results obtained employing the method of the present invention.

According to the present invention, the transport medium containing the HSV antigen to be assayed is contacted with a silanized glass solid support. There is no immunological binding between the HSV antigen to be determined with any substances that may be found on the silanized glass solid support. The transport medium containing the HSV antigen and the solid support are incubated for a sufficient period of time to permit the HSV antigen to adsorb onto the solid support.

Following the incubation period, the HSV antigen-coated solid support is washed with phosphate buffered saline containing 0.1% Tween 20® (PBST) to remove unadsorbed virus and tissue debris. The HSV antigen coated solid support is brought in contact with anti-herpes simplex virus monoclonal antibody, preferably anti-herpes simplex virus mouse monoclonal antibody; or anti-herpes simplex virus polyclonal antibody, preferably anti-herpes simplex virus rabbit polyclonal antibody. The resulting mixture is incubated for a sufficient period of time to allow formation of an immunocomplex on the solid support. The complex thus formed is washed with PBST to remove uncomplexed antibody.

The immunocomplex on the solid support is then treated with antiglobulin having an enzyme label conjugated thereon. The resulting mixture is incubated for a period of time sufficient to allow the formation of an antigen anti-HSV antibody: enzyme-labeled antiglobulin complex on the solid support. The complex thus formed is washed with PBST to remove unbound antiglobulin conjugate. A substrate that is catalyzed by the particular enzyme label conjugated to the antiglobulin is brought in contact with the antigen: anti-HSV antibody: enzyme-labeled antiglobulin complex to induce the enzyme activity, thereby causing the labeling effect. The substrate and the preceding complex is incu bated and the enzyme reaction is stopped with an appropriate stopping reagent. The reacted substrate is then read using, for example, a spectrophotometer.

The term " derivatized solid support" refers to an insoluble material having surface amino or alkyl groups and which is sorptive for the antigen. Known materials which may be derivatized include glass, borosilicate, sodalime, silica, cellulose, paper, and the like. The solid support may be derivatized with an aminotrialkoxysilane, having the structural formula:

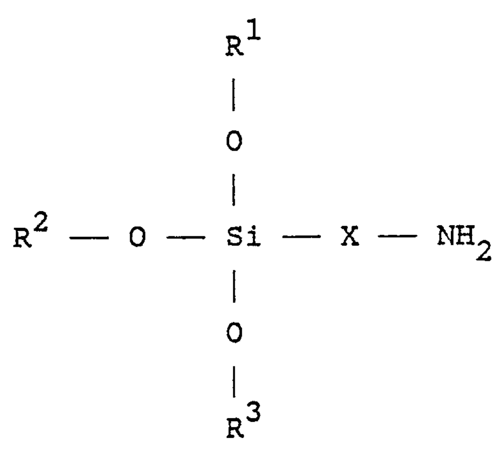

wherein $R^1$, $R^2$ and $R^3$ are independently alkyl groups each containing 1 to 10 carbon atoms, and X is either an alkyl, aryl, or aryloxyalkyl group containing 1 to 10 carbon atoms.

Among these aminotrialkoxysilanes, silanes wherein $R^1$, $R^2$, and $R^3$ are independently alkyl groups each containing 1 to 4 carbon atoms, and X is either an alkyl group, containing 1 to 4 carbon atoms, a phenyl group, or a phenoxyalkyl group, wherein the alkyl group contains 1 to 4 carbon atoms, are preferred. Particularly preferred is 3-aminopropyltriethoxysilane; and even more preferred is γ-(p-aminophenoxy)-triethoxysilane.

Alternatively, the solid support may be derivatized with a halotrialkylsilane, having the structural formula:

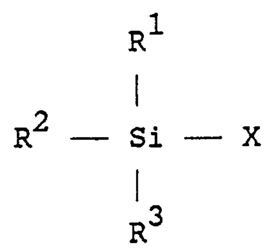

wherein $R^1$, $R^2$, and $R^3$ are independently alkyl groups each containing 1 to 10 carbon atoms, and X is a halogen atom. Among these halotrialkylsilanes, silanes wherein $R^1$, $R^2$, and $R^3$ are independently alkyl groups each containing 1 to 4 carbon atoms, are preferred. Particularly preferred, is chlorodimethylisopropylsilane.

Another alternative may also be where, the amino derivatized surface is quaternized with a variety of reagents, such as, for example, methyl iodide.

The solid support may be in the form of beads, tubes, strips, discs, microtitration plates, glass fiber filters, and the like; preferably glass beads; and even more preferably glass tubes.

The term "primary antibody" refers to a monoclonal or polyclonal antibody that is "immunoreactive" with one or more strains of herpesvirus antigens and is raised in a human or non-human species such as rabbit, goat, etc. The primary antibody, i.e., anti-herpes simplex antibody, is preferably, anti-HSV mouse monoclonal antibody, and even more preferably, anti-HSV rabbit polyclonal antibody, and is commercially available (Dako).

The term "secondary antibody" refers to an antiglobulin specific for a species in which the anti-herpes simplex antibody was derived and is raised in a non-human species such as goat, rabbit, etc.

The "primary antibody" may be directly labeled by conventional fluorescent dyes, enzymes or radioactive labels to permit determination of the bound HSV antigens on the solid support. Alternatively, the "secondary antibody" may be labeled with either fluorescent dyes, enzymes or radioactive labels by conventional methods. It is preferred to employ the "secondary antibody" labeled with an enzyme. Examples of enzymes include oxidases, hydrolases, lysases, decarboxylases, and the like. The enzyme, horseradish peroxidase is preferred.

Following the formation of the labeled complex, an enzyme substrate is reacted with the labeled complex and an enzyme determination is performed by conventional colorimetric, fluorometric, or spectrophotometric techniques. The substrate complementary to the preferred enzyme and, therefore preferred, is 3,3′,5,5′-tetramethylbenzidine (TMB).

For the purpose of giving those skilled in the art a better understanding of the present invention, the following illustrative, non-limiting examples are given.

## EXAMPLE 1

This example illustrates the preparation of silanized glass beads in accordance with the invention. The glass beads in this assay were sodalime glass silanized with 3-aminopropyltriethoxysilane. and are referred to herein as "3-aminopropyl glass." The nine-step preparation procedure is set forth below:

Step 1: In order to compare silanized and unsilanized glass, approximately 100 mg of 75-150μ glass beads (silanized or unsilanized) were placed on top of a porous disc inserted into the bottom of a plastic mini-column. A second disc was inserted firmly on top of the beads to hold them in place during the HSV assay. These columns generally use only gravity flow in the HSV assay but will perform as well with pressure or vacuum as applied to increase the flow rate. The void volume of this column was less than 0.2 mL. An excess volume (300 μL) of all reagents was used to ensure the previously pipetted agent was eliminated from the column.

Step 2: HSV antigen is prepared by the growth of the HSV A.T.C.C. No. VR-734 in HEp-2 cell A.T.C.C. No. CCL23. Noninfected Hep-2 cells were used as a negative control to determine assay background signal. 300 μL of sample (HSV-infected or noninfected HEp-2) was diluted in Minimal Essential Medium (MEM) (Gibco) containing 2% fetal calf serum and 1% NP-40® detergent (pH 7.5) and was pipetted into the column. Two concentrations of the diluted samples were prepared at 1:100 and 1: 500 in the MEM diluent.

Step 3: The HSV sample was incubated on the column for one minute at room temperature and washed with 2 mL of phosphate buffered saline containing 0.1% Tween 20®(PBST).

Step 4: 300 µL of rabbit-HSV antibody (Dako, diluted 1: 1000 in 90% fetal calf serum/phosphate buffered saline) was pipetted onto the column and incubated for ten minutes at room temperature.

Step 5: The column was washed with 5 mL of PBST and 300 µL of goat anti-rabbit-horseradish peroxidase conjugate (Bio Rad, diluted 1:3000 in 20% fetal calf serum/phosphate saline) was pipetted onto the column.

Step 6: The conjugate was incubated in the column for ten minutes at room temperature and the excess was washed out with 5 mL of PBST.

Step 7: 300 µL of TMB (3,3′,5,5′-tetramethylbenzidine) substrate was pipetted into each column. A substrate blank was made by running only substrate over a minicolumn containing 3-aminopropyl glass beads.

Step 8: The substrate was incubated for two minutes at room temperature. After incubation the columns were placed in glass collection tubes and the reaction was stopped with 300 µL of 2N HCl being pipetted into the column.

Step 9: 200 µL of reacted substrate from each collection tube was pipetted into a microtiter plate well and the plate was read on a spectrophotometer at 450 nm. The results are listed in Table I.

Tables IA and IB list the data pertaining to enhanced detection of herpes simplex virus on 3-aminopropyl glass beads. The data listed represents averages of each set of replications. Table IA shows the data for detecting HSV on nonderivatized glass. Table IB shows data for detecting HSV on 3-aminopropyl glass. The following description of each column pertains to both tables: Column 1 indicates the dilution of each respective sample. The negative control indicated therein, is non-infected HEp-2 cells. Column 2 shows the optical density (O.D.) reading at 450 nm for each sample. Column 3 shows the difference in optical density between the HSV sample and the negative control sample. Column 4 represents the ratio of the optical density of the HSV sample to the negative control. It is clear that immobilization of the HSV antigen on the 3-aminopropyl glass substantially increases the detection signal. This increase in signal is one indication of the increased sensitivity derived from the instant invention.

Results of Glass Bead Assay (Substrate Blank Has Been Subtracted From All O.D. 450 nm Readings)

Table IA

| | Sample Dilution | Sample (O.D.) | Difference (O.D.) | Ratio |
|---|---|---|---|---|
| Nonderivatized Glass | | | | |
| Neg. Control | (1:500) | 0.280 | | |
| HSV | (1:500) | 0.319 | 0.039 | 1.1 |
| Neg. Control | (1:100) | 0.240 | | |
| HSV | (1:100) | 0.528 | 0.288 | 2.2 |

Table IB

| | Sample Dilution | Sample (O.D.) | Difference (O.D.) | Ratio |
|---|---|---|---|---|
| 3-Aminopropyl Glass | | | | |
| Neg. Control | (1:500) | 0.099 | | |
| HSV | (1:500) | 0.352 | 0.253 | 3.6 |
| Neg. Control | (1:100) | 0.062 | | |
| HSV | (1:100) | 1.031 | 0.969 | 16.6 |

EXAMPLE 2

This example illustrates the preparation of silanized glass tubes in accordance with the present invention. The glass tubes were borosilicate, silanized with the same silanizing agent as in Example 1. The test procedure was as follows:

Step 1: As in Example 1, HSV and noninfected HEp-2 samples were diluted at 1: 125, 1: 250, and 1: 500 in MEM containing 2% fetal calf serum and 1% NP 40®.

Step 2: 300 µL of sample was pipetted into 12 X 75 3-aminopropyl glass tubes. The tubes were vertically rotated (180 rpm) for twenty minutes at room temperature.

Step 3: The tubes were washed twice with 1 mL of PBST.

Step 4: 500 µL of rabbit anti-HSV antibody, at a 1: 1000 dilution in 90% fetal calf serum and phosphate buffered saline was added to the tubes. The tubes were rotated (180 rpm) at room temperature for twenty minutes.

Step 5: The tubes were then washed three times with 1 mL of PBST.

Step 6: 300 µL of goat anti-rabbit-HRP conjugate, diluted 1: 3000 in 20% fetal calf serum-phosphate buffered saline. was pipetted into the tube. The tubes were rotated (180 rpm) at room temperature for twenty minutes.

Step 7: The tubes were washed three times with 1 mL of PBST.

Step 8: 300 µL of OPD substrate. consisting of 10 mg of o-phenylenediamine dihydrochloride in 10 mL of phosphate-citrate buffer (pH 5.2) with 0.03% hydrogen peroxide, was added to the tubes. A substrate blank was made by adding only substrate to a silanized glass tube. After incubating ten minutes at room temperature, the reaction was quenched with 100 µL of 2N sulfuric acid ($H_2SO_4$).

Step 9: 200 µL of the reacted substrate was pipetted into a microtiter plate well and the plate was read on the spectrophotometer at 492 nm. The results are listed in Table II.

Tables IIA and IIB show all data in accordance with Tables IA and IB, previously described, distinguishable by the type of support used, i.e., glass test tubes.

Results of Glass Test Tube Assay (Substrate Blank Has Been Subtracted From All O.D. 492 nm Readings)

Table IIA

| | Sample Dilution | Sample (O.D.) | Difference (O.D.) | Ratio |
|---|---|---|---|---|
| Nonderivatized Glass | | | | |
| Neg. Control | 1:500 | 0.023 | | |
| HSV | 1:500 | 0.410 | 0.387 | 17.8 |
| Neg. Control | 1:250 | 0.024 | | |
| HSV | 1:250 | 0.782 | 0.758 | 32.6 |
| Neg. Control | 1:125 | 0.028 | | |
| HSV | 1:125 | 1.013 | 0.985 | 36.1 |

Table IIB

| | Sample Dilution | Sample (O.D.) | Difference (O.D.) | Ratio |
|---|---|---|---|---|
| 3-Aminopropyl Glass | | | | |
| Neg. Control | 1:500 | 0.003 | | |
| HSV | 1:500 | 0.521 | 0.518 | 173.6 |
| Neg. Control | 1:250 | 0.016 | | |
| HSV | 1:250 | 0.921 | 0.905 | 57.5 |
| Neg. Control | 1:125 | 0.010 | | |
| HSV | 1:125 | 1.549 | 1.539 | 154.9 |

EXAMPLE 3

This example is a further illustration of the 3-aminopropyl silanized borosilicate glass tubes. Like Example 1, this example involves nine steps as set forth below:

Step 1: As in Example 1, HSV and noninfected HEp-2 samples were diluted at 1: 100 in MEM containing 2% fetal calf serum and 1% NP-40.

Step 2: 300 µL of sample was pipetted into 12 x 75 3-aminopropyl borosilicate glass tubes. The tubes were vertically rotated (180 rpm) for twenty minutes at room temperature.

Step 3: The tubes were washed once with 2 mL of PBST.

Step 4: 300 µL of rabbit anti-HSV antibody, at a 1:1000 dilution in 90% fetal calf serum and phosphate buffered saline was added to the tubes. The tubes were rotated (180 rpm) at room temperature for twenty minutes.

Step 5: The tubes were then washed once with 2 mL of PBST.

Step 6: 300 µL of goat anti-rabbit-HRP conjugate, diluted 1: 3000 in 20% fetal calf serum-phosphate buffered saline, was pipetted into the tube. The tubes were rotated (180 rpm) at room temperature for twenty minutes.

Step 7: The tubes were washed twice with 2 mL of PBST.

Step 8: 300 µL of TMB (3,3′,5,5′ tetramethylbenzidine) was added to the tubes. A substrate blank was made by adding only substrate to a silanized glass tube. After incubating two minutes at room temperature, the reaction was quenched with 100 µL of 2N hydrochloric acid (HCl).

Step 9: 300 µL of the reacted substrate was pipetted into a microtiter plate well and the plate was read on the spectrophotometer at 450 nm.

EXAMPLE 4

The procedure of Example 3 was repeated in every essential detail, except for the use of chlorodi methylisopropylsilane as the silanizing agent.

EXAMPLE 5

The procedure of Example 3 was repeated in every essential detail, except for the use of γ(p-aminophenoxy)propyltriethoxysilane as the silanizing agent.

Table III lists the results of Examples 3, 4 and 5. It is clear that immobilization of the HSV antigen on either the chlorodimethylisopropyl/borosilicate glass or p-aminophenyl/borosilicate glass significantly increases the detection signal. Variations in the 3-aminopropyl/borosilicate glass tubes data, when compared with the previous examples, are attributable to irregularities in the silanization procedure and the borosilicate glass.

## Table III

Results of Borosilicate Glass Tubes (Substrate Blank Has Been Subtracted From All O.D. 450 nm Readings)

| | Sample Dilution | Sample (O.D.) | Diff.* (O.D.) | Ratio |
|---|---|---|---|---|
| Nonderivatized Glass | | | | |
| Neg. Control | (1:100) | 0.055 | | |
| HSV | (1:100) | 1.407 | 1.352 | 25.7 |
| 3-Aminopropyltriethoxysilane | | | | |
| Neg. Control | (1:100) | 0.050 | | |
| HSV | (1:100) | 1.082 | 1.032 | 21.8 |
| Chlorodimethylisopropylsilane | | | | |
| Neg. Control | (1:100) | 0.048 | | |
| HSV | (1:100) | 1.477 | 1.428 | 30.8 |
| p-Aminophenylpropyltriethoxysilane | | | | |
| Neg. Control | (1:100) | 0.043 | | |
| HSV | (1:100) | 1.651 | 1.608 | 38.4 |

*Difference

### EXAMPLE 6

This example illustrates 3-aminopropyl/sodalime glass tubes. The test procedure was a follows:

Step 1: As in Example 1, HSV and noninfected HEp-2 samples were diluted at 1: 100 in MEM containing 2% fetal calf serum and 1% NP-40.

Step 2: 300 µL of sample was pipetted into 12 x 75 3-aminopropyl sodalime glass tubes. The tubes were vertically rotated (180 rpm) for twenty minutes at room temperature.

Step 3: The tubes were washed once with 2 mL of PBST.

Step 4: 300 µL of rabbit anti-HSV antibody, at a 1:1000 dilution in 90% fetal calf serum and phosphate buffered saline was added to the tubes. The tubes were rotated (180 rpm) at room temperature for twenty minutes.

Step 7: The tubes were washed twice with 2 mL of PBST. Step 8: 300 µL of TMB (3,3′,5,5′ tetramethylbenzidine) substrate was added to the tubes. A substrate blank was made by adding only substrate to a silanized glass tube. After incubating two minutes at room temperature, the reaction was quenched with 100 µL of 2N hydrochloric acid (HCl).

Step 9: 300 µL of the reacted substrate was pipetted into a microtiter plate well and the plate was read on the spectrophotometer at 450 nm. The results are listed in Table IV.

## EXAMPLE 7

The procedure of Example 6 was repeated in every essential detail. except for the use of chlorodimethylisopropylsilane as the silanizing agent.

## EXAMPLE 8

The procedure of Example 6 was repeated in every essential detail, except for the use of γ(p-aminophenoxy)propyltriethoxysilane as the silanizing agent.

Table IV lists the results of Examples 6, 7 and 8.

### Table IV

Results of Sodalime Glass Tubes
(Substrate Blank Has Been Subtracted
From All O.D. 450 nm Readings)

| | Sample Dilution | Sample (O.D.) | Diff.* (O.D.) | Ratio |
|---|---|---|---|---|
| Nonderivatized Glass | | | | |
| Neg. Control | (1:100) | 0.088 | | |
| HSV | (1:100) | 0.911 | 0.824 | 10.4 |
| 3-Aminopropyltriethoxysilane | | | | |
| Neg. Control | (1:100) | 0.062 | | |
| HSV | (1:100) | 1.111 | 1.049 | 17.9 |
| Chlorodimethylisopropylsilane | | | | |
| Neg. Control | (1:100) | 0.068 | | |
| HSV | (1:100) | 1.182 | 1.114 | 17.4 |
| p-Aminophenylpropyltriethoxysilane | | | | |
| Neg. Control | (1:100) | 0.061 | | |
| HSV | (1:100) | 0.975 | 0.914 | 16.0 |

*Difference

EXAMPLE 9

This example illustrates 3-aminopropyl borosilicate glass tubes, utilized in conjunction with actual patient samples.

The positive and negative samples were obtained from clinical laboratories and have been previously tested by culture techniques known by those skilled in the art. The negative samples were received with the lesion swabs submerged in a transport medium contained in the sample tubes. The tubes were vortexed and the swabs were expressed in the tubes. The positive samples were delivered without swabs. All samples were pipetted neat into the assay test tubes containing 3 µL NP-40®. The test procedure was as follows:

Step 1: As in Example 1, HSV and noninfected HEp-2 control samples were diluted at 1:300 in MEM containing 2% fetal calf serum and 1% NP-40®.

Step 2: 300 µL of sample was pipetted into 12 x 75 3-aminopropyl glass tubes. The tubes were vertically rotated (180 rpm) for twenty minutes at room temperature.

Step 3: The tubes were washed twice with 2 mL of PBST.

Step 4: 300 µL of rabbit anti-HSV antibody (DAKO), at a 1:1000 dilution, or at a 1:1429 dilution (BioRad), in 50% fetal calf serum and phosphate buffered saline was added to the tubes. The tubes were rotated (180 rpm) at room temperature for twenty minutes.

Step 5: The tubes were then washed three times with 2 mL of PBST.

Step 6: 300 µL of goat anti-rabbit-HRP conjugate, diluted 1:3000 in 20% fetal calf serum-phosphate buffered saline, was pipetted into the tube. The tubes were rotated (180 rpm) at room temperature for twenty minutes.

Step 7: The tubes were washed three times with 2 mL of PBST.

Step 8: 300 µL of OPD substrate, consisting of 10 mg of o-phenylenediamine dihydrochloride in 10 mL of phos phate citrate buffer (pH 5.2) with 0.03% hydrogen peroxide, was added to the tubes. A substrate blank was made by adding only substrate to a silanized glass tube. After incubating ten minutes at room temperature, the reaction was quenched with 100 µL of 2N sulfuric acid ($H_2SO_4$).

Step 9: 200 µL of the reacted substrate was pipetted into a microtiter plate well and the plate was read on the spectrophotometer at 450 nm.

The results are listed in Tabel V. The data listed represents the single ssamples only. The far left hand column lists each patient sample tested, further divided into positive, negative and control samples, respectively. The remaining columns, labeled horizontally from left to right, list the data obtained from DAKO rabbit anti-HSV antibody and from BioRad rabbit anti-HSV antibody -- each further divided into data obtained on nonderivatized glass and 3-aminopropyl glass, respectively.

It is clear that immobilization of the clinically obtained HSV antigen on the 3-aminopropyl borosilicate glass substantially increases the detection signal of the positive samples and decreases the non-specific signal of the negative samples. This increase in signal is just another indication of the increased sensitivity derived from the present invention.

Table V

Results of Patient Samples On
Silanized Borosilicate Tubes
(Substrate Blank Has Been Subtracted
From All O.D. 450 nm Readings)

| | Sample Dilution | Dako Ab/ Non. Gl. | Dako Ab/ Sil. Gl. | B-R Ab/ Non. Gl. | B-R Ab/ Sil. Gl. |
|---|---|---|---|---|---|
| Positive Samples | | | | | |
| HS-A006 | | 0.032 | 0.058 | 0.159 | 0.262 |
| HS-A033 | | 0.198 | 0.313 | 0.261 | 0.304 |
| HS-A021 | | 0.072 | 0.127 | 0.165 | 0.321 |
| HS-A034 | | 0.007 | 0.046 | 0.110 | 0.228 |
| Negative Samples | | | | | |
| HS-A052 | | 0.025 | 0.000 | 0.140 | 0.036 |
| HS-A059 | | 0.040 | 0.032 | 0.109 | 0.021 |
| HS-A056 | | 0.019 | 0.011 | 0.053 | 0.000 |
| HS-A060 | | 0.057 | 0.028 | 0.096 | 0.028 |
| Controls | | | | | |
| Neg. Control | (1:300) | 0.022 | 0.033 | 0.046 | 0.000 |
| HSV | (1:300) | 1.366 | 1.303 | 1.222 | 0.790 |

## Claims

1. A method for detecting herpesvirus in a sample comprising:

(a) subjecting said sample to lysing conditions to disrupt any herpesvirus present therein;

(b) contacting said sample with a silane-derivatized solid support to immobilize thereon by hydrophobic interaction alone any herpesvirus so disrupted;

(c) contacting the product of step (b) with a primary antibody specific for said disrupted virus to form an immunocomplex;

(d) contacting the product of step (c) with a secondary antibody, said secondary antibody having appended thereto a label, to form a labeled immunocomplex; and

(e) detecting said labeled immunocomplex.

2. A method according to claim 1, wherein said silane-derivatized solid support is a solid support treated with an aminotrialkoxysilane having the structural formula:

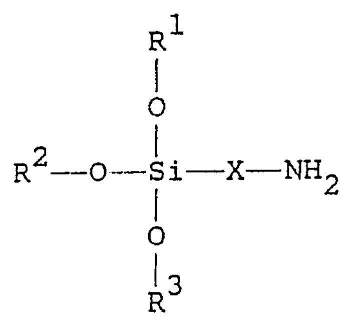

wherein $R^1$, $R^2$, and $R^3$ are independently alkyl groups each containing 1 to 10 carbon atoms, and X is either an alkyl, aryl, or aryloxyalkyl group containing up to 10 carbon atoms.

3. A method according to claim 2, wherein said aminotrialkoxysilane is a member selected from the group consisting of 3-aminopropyltriethoxysilane and γ(p-aminophenoxy)propyltriethoxysilane.

4. A method according to claim 1, wherein said silane derivatized solid support is a solid support treated with a halotrialkylsilane having the general structural formula:

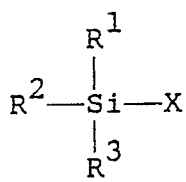

wherein, $R^1$, $R^2$, and $R^3$ are independently alkyl groups each containing 1 to 10 carbon atoms, and X is a halogen atom.

5. A method according to claim 4, wherein said halotrialkylsilane is chlorodimethylisopropylsilane.

6. A method according to claim 1, wherein said silane-derivatized solid support is sodalime glass or borosilicate glass.

7. A method according to claim 1, wherein step (a) comprises contacting said sample with a detergent selected from the group consisting of nonionic and zwitterionic detergents.

8. A method according to claim 1, wherein step (a) comprises contacting said sample with a member selected from the group consisting of urea, potassium thiocyanate and guanidine hydrochloride.

9. A method according to claim 1, wherein said secondary antibody of step (d) is an antiglobulin.

10. A method according to claim 1, wherein said label appended to said secondary antibody of step (d) is an enzyme.

European Patent Office

# EUROPEAN SEARCH REPORT

Application Number

EP 89 10 2827

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | BIOLOGICAL ABSTRACTS, vol. 76, 1983, abstract no. 33928, Biological Abstracts Inc., Philadelphia, PA, US; D.O. MATSON et al.: "A micro solid-phase radioimmunoassay for detection of herpesvirus type-specific antibody: Parameters involved in standardization", & J. VIROL METHODS 6(2): 71-84. 1983<br>* Abstract *<br>--- | 1,2,9, 10 | G 01 N 33/569<br>G 01 N 33/552 |
| X | AU-A- 554 097 (P.J. BAIRD)<br>* Page 3, line 5 - page 4, line 23; page 5, line 30 - page 6, line 4; page 9, lines 8-11; page 17, line 2 - page 19, line 27 *<br>--- | 1-10 | |
| Y | CHEMICAL ABSTRACTS, vol. 86, 1977, page 410, abstract no. 187415e, Columbus, Ohio, US; K.O.K. KALIMO et al.: "Solid-phase radioimmunoassay of human immunoglobulin M and immunoglobulin G antibodies against herpes simplex virus type 1 capsid, envelope, and excreted antigens", & INFECT. IMMUN. 1977, 15(3), 883-9<br>--- | 1-10 | TECHNICAL FIELDS SEARCHED (Int. Cl.4)<br>G 01 N |
| Y | DE-A-2 042 976 (CORNING GLASS WORKS)<br>* Page 6, lines 10-13; page 14, lines 5-9; page 30, lines 9-18; page 31, lines 7-17 *<br>--- -/- | 1-10 | |

The present search report has been drawn up for all claims

| Place of search | Date of completion of the search | Examiner |
|---|---|---|
| THE HAGUE | 25-07-1989 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 89 10 2827

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | METHODS IN ENZYMOLOGY, vol. 34, 1974, pages 59-72, US; H.H. WEETALL et al.: "Porous glass for affinity chromatography applications" * Page 59, lines 17-19; page 63, line 15 - page 72, line 5 * --- | 2-6 | |
| X | US-A-4 588 680 (D.J. BUCHER et al.) * Column 3, lines 17-34 * --- | 7,8 | |
| X | GB-A-2 014 727 (SANYO CHEMICAL INDUSTRIES) * Page 1, line 56 - page 2, line 2; page 3, lines 22-33; page 9, line 40 - page 11, line 19 * ----- | 2-5 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 25-07-1989 | VAN BOHEMEN C.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)